Europäisches Patentamt

European Patent Office

Office européen des brevets

(11) Publication number: **0 406 937 A2**

# EUROPEAN PATENT APPLICATION

(21) Application number: 90201671.6

(51) Int. Cl.⁵: **C12N 15/10**, C07H 21/04

(22) Date of filing: 23.06.90

(30) Priority: 30.06.89 US 373560
28.07.89 US 387054

(43) Date of publication of application:
09.01.91 Bulletin 91/02

(84) Designated Contracting States:
DE FR GB

(71) Applicant: EASTMAN KODAK COMPANY
343 State Street
Rochester, New York 14650(US)

(72) Inventor: Ciccarelli, Richard Benjamin,
EASTMAN KODAK COMP.
Patent Department, 343 State Street
Rochester, New York 14650(US)
Inventor: Oakes, Fred Terry, c/o EASTMAN
KODAK COMPANY
Patent Department, 343 State Street
Rochester, New York 14650(US)

(74) Representative: Nunney, Ronald Frederick
Adolphe et al
Kodak Limited Patent Department Headstone
Drive
Harrow Middlesex HA1 4TY(GB)

(54) In vitro gene synthesis.

(57) A method of making double-stranded DNA sequences involving application of polymerase chain reactions to a mixture of crude oligonucleotide broths of the top and bottom strands of a targeted double-stranded DNA sequence is disclosed.

EP 0 406 937 A2

## IN VITRO GENE SYNTHESIS

This invention relates to a method of making double-stranded DNA sequences such as genes.

Recent advances in DNA chemistry, instrumentation and enzymology have made in vitro synthesis of genes from specific DNA oligonucleotides possible. Genes synthesized in vitro (synthetic genes) have several advantages over their cloned natural counterparts. They are usually more compact than eucaryotic genes since they contain no intron regions. Also, they do not contain large DNA flanking sequences which are commonly present when natural genes are isolated from cells. In addition, due to the degeneracy in the genetic code, amino acid codons can be chosen to match the optimal codons utilized by a selected cloning host, leading to higher levels of gene and/or protein expression. Unique DNA restriction enzyme sites can be designed into the gene, or added to the ends of the gene. The latter allows cloning of the gene into an expression vector. The former allows screening of the gene after cloning. Genes synthesized in vitro are used for the production of important commercial and pharmaceutical proteins.

The synthetic gene can be designed to code for a protein sequence that exactly matches the protein sequence coded by the "native" gene, or the synthetic gene can code for altered (mutant) proteins which might have advantages over the native proteins such as increased stability. In addition, synthetic genes can be designed which code for entirely novel proteins or novel combinations or protein parts (domains).

Various strategies for chemically synthesizing genes in vitro have been used to construct a wide variety of genes. Most of the chemical synthesis strategies involve making oligonucleotides comprising both DNA strands of the gene and ultimately joining them together to give the complete double-stranded gene.

The most widely used strategy involves chemical synthesis of both strands in a series of short, overlapping oligonucleotides (each approximately 40 nucleotides in length) which, when annealed and joined (ligated) together, comprise the complete gene. The problem is that to chemically synthesize a relatively small gene of 400 base pairs (bp), this strategy requires that a) a total of 800 bases (400 x 2 strands) be synthesized and b) 20 oligonucleotides of up to 40 bases each be separately synthesized and separately purified by gel electrophoresis and/or high pressure liquid chromatography (HPLC).

Following purification, the oligonucleotides are phosphorylated, mixed, heated and allowed to anneal to their homologous counterparts. DNA ligase is added to the mixture to ligate the double-stranded DNA segments to form the complete gene. The ligation reaction should be characterized by gel electrophoresis in order to assure that a piece of the expected size (i.e. 400 bp) has resulted.

However, due to the complexity of the oligonucleotide mixture in the annealing and ligation steps, obtaining a piece of the correct size does not assure that the new gene has the correct sequence. Therefore, the synthetic gene is cloned into a plasmid for characterization by DNA sequencing. Another problem is that if the sequence is not correct, the gene must be corrected or the entire process must be carried out again.

The object of the present invention is to provide a method for making double-stranded DNA having at least 400 base pairs while eliminating the need a) to make over 20 different oligonucleotides and b) repeat the entire chemical synthesis when there is a sequence error in snythetic double-stranded DNA.

Figure 1 shows schematically how the polymerase chain reaction (PCR) is used to achieve construction of double-stranded DNA sequences.

Figure 2 shows the DNA sequence of the native and synthetic HIV-1 REV gene used to illustrate the method of this invention.

The present invention meets the foregoing objective by providing a method of making double-stranded DNA sequences (dsDNA), such as genes, characterized by the steps of:

A) preparing first and second synthesis broths, each comprising

    i) DNA oligonucleotides of a defined nucleotide length and failure sequences thereof; or

    ii) the failure sequences only; wherein the first synthesis broth comprises DNA oligonucleotides that include the top strand or failure sequences thereof of the targeted double-stranded DNA sequence and the second synthesis broth comprises DNA oligonucleotides that include the bottom strand or failure sequences thereof of the targeted DNA sequence;

B) mixing the first and second synthesis broths together;

C) adding DNA oligonucleotide primers that are complementary to the 3′ ends of the DNA oligonucleotides of A);

D) applying polymerase chain reaction (PCR) procedures to the mixture of C) thereby amplifying exponentially the concentration of the target double-stranded DNA sequence;

E) isolating the target double-stranded DNA sequence;

F) inserting the target double-stranded DNA sequence into a plasmid having a phage origin of replication;

G) sequencing the target double-stranded DNA sequence to identify mutations; and

H) repairing the mutations by site-directed mutagenesis.

During the synthesis of oligonucleotides of a defined length, many oligonucleotides having less than the defined length are also synthesized. When the synthesis is carried out for the top and bottom strands of a targeted dsDNA sequence, those oligonucleotides of the top and bottom strands that a) overlap, b) have the end DNA sequence of the top or bottom strand and c) have less than the desired length are defined herein as failure sequences.

There are several advantages provided by this method.

Fewer oligonucleotides need be synthesized. For a 400 bp dsDNA only 2 long oligonucleotides corresponding to each strand of the gene and 2 short DNA synthesis primers need be synthesized. They are used directly from the crude synthesis broth after desalting.

The targeted dsDNA sequence can be made with preferred amino acid codons for increased expression, and convenient restriction sites for cloning and characterization by DNA sequencing.

The targeted dsDNA can be completely amplified in Just a few hours.

Errors in construction can be directly corrected by mutagenesis following insertion of the target dsDNA sequence into a plasmid containing a phage origin of replication.

A gene can be constructed out of translational frame with the plasmid gene expression system to avoid problems which might arise if the synthetic gene product (protein) is toxic to the host cell. The gene can later be put into frame by mutagenesis.

A gene can be synthesized directly as an in-frame gene fusion with a second gene, creating novel chimeric genes.

The synthetic gene can be directly altered by site-directed mutagenesis without subcloning steps, making protein structure:function studies more convenient to perform.

Parts of genes and exon regions of genes, corresponding to functional regions of proteins or domains of proteins, are conveniently synthesized.

Genes or parts of genes or chimeric genes which code for proteins which can be used for biochemicals, enzymes, pharmaceuticals, vaccines, diagnostic reagents or agriculturals can be conveniently synthesized.

## Details of the Invention

Essentially, the method of the present invention comprises preparing targeted dsDNA sequences, such as synthetic genes, by PCR amplification of synthesized oligonucleotides and oligonucleotide failure sequences corresponding to the top ($5'\rightarrow3'$) and bottom ($3'\rightarrow5'$) strands of the gene, and site-directed mutagenesis to correct mutations. The method is simplified by inclusion of synthetic DNA sequences adjacent to each end of the targeted DNA sequences prior to amplification. This allows cloning (inserting) of the final double-stranded sequence (e.g. the gene) into a plasmid containing a phage origin of replication [e.g., F1(IG)] in order to carry out site-directed mutagenesis and DNA sequence analysis.

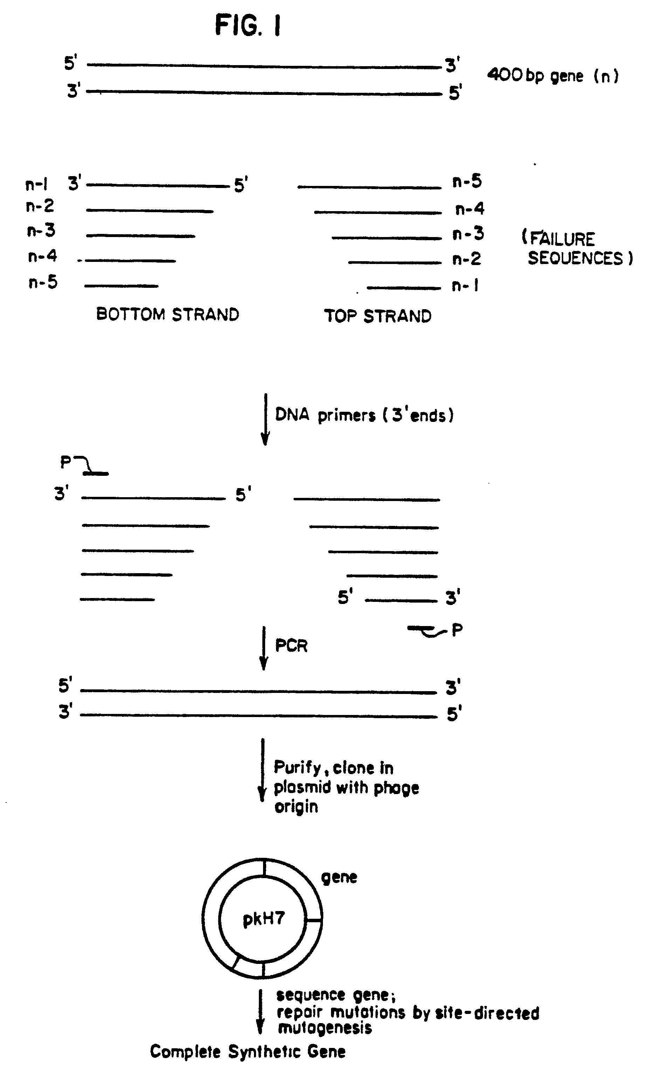

PCR amplification is carried out on crude synthesis broths containing the oligonucleotides and oligonucleotide failure sequences corresponding to the top ($5'\rightarrow3'$) and bottom ($3'\rightarrow5'$) strands which partially overlap (base pair to) each other. Such partially overlapping DNA sequences are present in large quantity as by-products in the crude synthesis broths generated from an automated DNA synthesizer. These partially overlapping DNA sequences are defined as failure sequences herein. For example, (see Figure 1) the synthesis of the top strand ($5'\rightarrow3'$) of a 400 base gene (n = 400) on an automated DNA synthesizer results in a crude synthesis broth that may comprise the full oligonucleotide of n bases, as well as n-1, n-2, n-3... failure sequences.

Each base addition reaction has a yield of approximately 98% (herein known as the coupling efficiency). Since the coupling efficiency at each step is less than 100%, the final yield of DNA oligonucleotide full length product will be less than 100% of the total DNA in the synthesis broth. The remainder of the DNA in the synthesis broth is thus comprised of the failure sequences.

Such failure sequences actually comprise the majority of DNA product (on a Molar basis) in the crude synthesis broth. Furthermore, the full length DNA (n) and failure sequences (n-1, n-2, n-3, etc.) all have a common $3'$-end. This is because the first base added to initiate DNA synthesis on an automated DNA synthesizer is at the $3'$ end of the required sequence. Subsequent base additions occur sequentially in the $3'\rightarrow5'$ direction. Chain growth occurs sequentially from the $3'$ end of the oligonucleotide.

Thus, the final crude synthesis broth may comprise none or only a small quantity of the full length oligonucleotide (n) and does comprise failure sequences (overlapping nucleotides of less than full length) all with common $3'$ ends. This, is also the situation for the synthesis of the full length oligonucleotide (n') corresponding to the bottom strand (3'→5') of the gene. The crude synthesis broth comprises 3' ends.

The method now requires the mixing together of the two crude synthesis broths. In addition, two short DNA sequences (primers) are added which match the common 3' ends of the top strand (n) and failure sequences (n-1, n-2...) and the bottom strand (n') and failure sequences (n'-1, n'-2, n'-3...). See p in Figure 1.

The polymerase chain reaction is now carried out. The method of this invention takes advantage of the fact that the PCR product of the top strand failure sequences overlap with the PCR product of the bottom strand failure sequences. When this situation occurs, all of the information (base sequence) necessary for the amplification of the entire targeted DNA sequence is present even if none of the full length oligonucleotides is present in the broth. Subsequent cycles of PCR ultimately amplify the entire gene sequence. In practice, the first three cycles of PCR amplification are actually carried out at very long extension times (up to 30 minutes instead of the usual 1 minute) in order to begin the PCR chain reaction.

For example, synthesis of a 400 base pair double-stranded sequence is carried out as follows:

Crude DNA oligonucleotide synthesis broths are prepared which comprise the top strand (n = 400) and top strand failure sequences 399 (n-1), 398 (n-2), 397 (n-3), and which comprise the bottom strand (n' = 400) and bottom strand failure sequences 399 (n'-1), 398 (n'-2), 397 (n'-3). Mixing together of the two synthesis broths results in partially overlapping sequences. For example, sequences of the top strand having a length greater than n12 would overlap with the bottom strand having sequences greater than n'-L bases in length (L') such that there is sufficient complementarity in the overlap to allow PCR extension reactions. The addition of two separate DNA primer sequences (15 - 20 base sequence) which base pair to the 3' ends of the top strand sequences and also to the bottom strand sequences is now sufficient to start the polymerase chain reaction.

In practice, the quantity of the DNA oligonucleotides, (n...n-199) and (n'...n'-199), which actually overlap can be extremely limited since the PCR can amplify a single molecule of double-stranded DNA. Again, the broths need not contain any full length oligonucleotides for the method of this invention to work. The first round of PCR would now produce DNA products from the top and bottom strands which would be used in subsequent rounds of PCR to give the entire 400 bp gene sequence.

Convenient DNA sequences corresponding to restriction enzyme cleavage sites are built into the gene sequence and are also amplified with the gene by PCR. Such sites allow for the insertion of the amplified gene directly into a plasmid. The plasmid contains a phage origin of replication. This allows convenient DNA sequence analysis of the PCR product. This also allows for convenient site-directed mutagenesis, in order to correct any mutations generated during oligonucleotide synthesis or during the PCR amplification steps.

Double-stranded DNA sequences (e.g. genes) of larger than 400 base pairs are also conveniently synthesized directly by the method, or indirectly by carrying out the method in a stepwise manner. For example, the synthesis of a much larger DNA sequence would be carried out by first making a series of portions of the large sequence according to steps A-H. These portions could then be ligated (joined) by the enzyme DNA ligase to give the full length sequence. Alternatively, these portions could be designed to overlap each other in sequence. The entire large sequence could then be amplified by PCR.

Accordingly, targeted double-stranded DNA sequences having greater than 400 bp are prepared by:

A) preparing a series of first and second synthesis broths, each comprising;

    i) DNA oligonucleotides of a defined nucleotide length and failure sequences thereof; or

    ii) the failure sequences only; wherein

        a) the first broth of each serial member of the series comprises DNA oligonucleotides equivalent to a defined portion of the top strand or failure sequences thereof of the targeted double-stranded DNA sequence;

        b) the second broth in each serial member of the series compriges DNA oligonucleotides equivalent to a defined portion of the bottom strand or failure sequences thereof of the targeted double-stranded DNA sequence; and

        c) the entire series, taken together, is equivalent to the entire targeted double-stranded DNA sequence;

B) separately mixing the first and second synthesis broths of each member of the series together;

C) adding to each mixture of the series DNA oligonucleotide primers that are complementary to the 3' ends of the DNA oligonucleotides in each mixture;

D) applying polymerase chain reaction procedures to each mixture thereby amplifying exponentially the concentration of the targeted double-stranded DNA sequence equivalents in each mixture;

E) isolating the targeted double-stranded DNA sequence equivalents from each mixture in the series;

F) cloning each of the equivalents in a cloning vector having a phage origin of replication;

G) sequencing each equivalent to identify any mutations;

H) repairing any mutations by site-directed mutagenesis;

I) removing each equivalent of the series from the cloning vector;

J) ligating the entire series of equivalents together to form the targeted double-stranded DNA sequence having more than 400 base pairs.

The oligo comprising the targeted dsDNA sequence and failure sequences thereof and primers used to amplify the properly assembled fragments may be prepared using any suitable method, such as, for example, the phosphite triester, the phosphotriester and phosphodiester methods of the prior art or automated embodiments. In one such automated method diethylphosphoramidites are used as starting materials. They may be synthesized as described by L.J. mcBride et al, Tetrahedron Letters , 24:25 (1983), U.S. Patent 4,458,066 and Beaucage et al, Tetrahedron Letters (1981), 22:1859-1862. A method for synthesizing oligo on a modified solid support is also described in U.S. Patent 4,458,066.

Polymerase Chain Reaction (PCR)

The small quantity of large oligonucleotides is amplified using a variation of a polymerase chain reaction described in U.S. Patents 4,683,195 and 4,683,202. The reaction uses primers and polymerization agents.

The PCR technique is conceptually a very simple method for amplifying nucleic acids. It somewhat mimics the natural DNA replication process in that the number of DNA molecules generated by the Polymerase Chain Reaction doubles after each cycle, in a way similar to in vivo DNA replication.

Tne method is based on the repetition of a set of three steps, all conducted in succession under somewhat different and controlled temperature conditions. The steps are denaturation, annealing and primer extension.

The term "primer" as used herein refers to an oligonucleotide sequence that provides an end to which polymerization agents, such as DNA polymerase including Thermus aquaticus DNA polymerase, can add nucleotides that are complementary to a nucleotide sequence (template) to which the primer is annealed. The addition occurs in the presence of deoxyribonucleosides triphosphates (dNTPs), at a suitable temperature and pH. The primer is single-stranded or a mixture of single-stranded and double-stranded oligonucleotides. The primer must be sufficiently long to prime the synthesis of extension products in the presence of the agent for polymerization. The exact lengths of the primers will depend on many factors, including the temperature and source of primer. For example, depending on the · complexity of the target sequence, the oligo primer typically contains 15-25 or more nucleotides, although it may contain fewer nucleotides. Short primer molecules generally require cooler temperatures to form sufficiently stable hybrid complexes with template.

The primers are selected to be "substantially" complementary to their templates. This means that the primers are sufficiently complementary to hybridize with their templates. Therefore, the primer sequence need not reflect the exact sequence of the template. For example, a non-complementary nucleotide fragment may be attached to the $5'$ end of the primer, with the remainder of the primer sequence being complementary to the strand. Alternatively, non-complementary bases of longer sequences can be interspersed into the primer, provided that the primer sequence has sufficient complementarity with the sequence of the strand to be amplified to hybridize therewith and thereby form a template for synthesis of the extension product of the other primer.

The primers are prepared using the same methods used to prepare the oligo fragments from which the genes are prepared.

Denaturation

After isolation of the desired double-stranded DNA sequence, it is necessary to separate the strands so they can each be used individually as templates. Separation of the strands can occur in a separate step or simultaneously with the synthesis of the primer extension products. This strand separation can be accomplished by any suitable denaturing method including physical, chemical or enzymatic means.

One physical method of separating the strands of the nucleic acid involves heating the nucleic acid until it is completely (>99%) denatured. Typical heat denaturation may involve temperature ranging from 800 to 105° C. for times ranging from 1 to 10 minutes. Strand separation may also be induced by an enzyme from the class of enzymes known as helicases. The reaction conditions suitable for separating the strands of nucleic acids with helicases are described by Cold Spring Harbor Symposia on Quantitative Biology , Vo. XLIII "DNA: Replication and Recombination" (New ark: Cold Spring Harber Laboratory, 1978) B. Kuhn et al, "DNA Belicase", pp. 63-67, and techniques for using RecA are reviewed in C. Radding, Ann. Rev. Genetics, 16:405-37 (1982).

The two strands, once dissociated, will remain free in solution until the temperature is lowered sufficiently to allow annealing.

Annealing of Extension primers

When the complementary strands of the nucleic acid or acids are separated, the strands are ready to be used as a templates for the synthesis of additional nucleic acid strands.

The extension primers are the pair of synthetic oligos which anneal to sites on the template flanking the region to be amplified. Each primer in the pair will anneal to only one of the strands of DNA. The sequence of the primers is determined by the sequence of the DNA template, at the boundaries of the region to be amplified. Since the primers anneal to opposite strands, they can be viewed as having their 3' ends facing each other. Typically, the primers have different sequences and are not complementary to each other.

Generally the primers are present in large excess over the DNA template. This favors the formation of the primer-template complex over the reassociation of the two DNA strands, at the primers' annealing sites, when the temperature is lowered.

Primer Extension (Amplification)

The third step in the procedure is the DNA polymerase-mediated $(5' \rightarrow 3')$ extension of the primer. The conditions under which the extension step is conducted depend directly on the type of DNA pojymerase used. Through this process, the extension primers will become incorporated into the amplification product.

In the PCR technique, the typical set of three steps (i.e., denaturation, annealing, extension) is referred to as a cycle. As taught by U.S. Patents 4,683,195 and 4,683,202, the technique is carried out on long pieces of DNA. The amplified product of interest is referred to as "short product", which is defined as the region comprised between the 5' ends of the extension primers. Since the primers have well-defined sequences, the short product will have discrete ends, corresponding to the primers' sequences. As the number of cycles increases, the short product will rapidly become the predominant template to which the extension primers will anneal. In theory, the amount of short product will double after each cycle, leading to an exponential accumulation.

The actual primer extension and amplification is carried out as follows. The deoxyribonucleoside triphosphates, dATP dCTP, dGTP and dTTP are also added to the synthesis mixture in adequate amounts and the resulting solution is heated to 90°-100° C for 1 to 10 minutes, preferably from 1 to 4 minutes. After this heating period the solution is allowed to cool to 20°-55° C, which is preferable for primer hybridization. An agent for polymerization is added to the cooled mixture. The reaction is allowed to occur under conditions known in the art. This synthesis reaction may occur at room temperature up to a temperature above which the agent for polymerization no longer functions efficiently. Thus, for example, if DNA polymerase I is used as the agent for polymerization, the temperature is generally no greater than 45° C.

The agent for polymerization may be any compound or system which will function to accomplish the synthesis of primer extension products including enzymes. Suitable enzymes for this purpose include, for example, E. coli DNA polymerase I, Klenow fragment of E. coli DNA polymerase I, T4 DNA polymerase, other available DNA polymerases, reverse transcriptase, and other enzymes, including heat-stable enzymes, which will facilitate combination of the nucleotides in the proper manner to form the primer extension products which are complementary to each nucleic acid strand. At higher temperatures up to 65 to 75° C, a thermostable polymerization agent, such as Thermus aquaticus polymerase, DNA polymerase is used. Generally, the synthesis will be initiated at the 3' end of each primer and proceed in the 5' direction along the template strand, until synthesis terminates.

There may be agents, however, which initiate synthesis at the 5' end and proceed in the other direction, using the same process as described above.

The newly synthesized strand and its template form a double-stranded molecule which is used in the succeeding steps of the process. The succeeding steps of the process again involve repeated application of the set of three steps (denaturation, annealing and primer extension).

New nucleic acid is synthesized on the single-stranded molecules. Additional inducing agent, nucleotides and primers may be added if necessary for the reaction to proceed under the conditions prescribed above. Again, the synthesis will be initiated at one end of the oligo primers and will proceed along the single strands of the template to produce additional nucleic acid. After this step, half of the extension product will consist of the specific nucleic acid sequence bounded by the two primers.

The polymerase chain reaction (PCR) can be performed in a step-wise fashion where after each step new reagents are added. Or it can be performed simultaneously, where all reagents are added at the initial step, or partially step-wise and partially simultaneous, where fresh reagent is added after a given number of steps.

If a method of strand separation, such as heat, is employed which will inactivate the agent for polymerization, as in the case of a heat-stable polymerization agent, then it is necessary to replenish the agent after every strand separation step.

The simultaneous method may be utilized when a number of purified components, including an enzymatic means such as helicase, is used for the strand separation step. In the simultaneous procedure, the reaction mixture may contain, in addition to the nucleic acid strand(s) containing the desired sequence, (1) the strand-separating enzyme (e.g., helicase), (2) an appropriate energy source for the strand-separating enzyme, such as ATP, (3) the four deoxyribonucleotides, (4) the oligonucleotide primers in molar excess, and (5) the polymerization agent.

If heat is used for denaturation in a simultaneous process, a heat-stable polymerization agent such as the thermostable polymerase referred to above may be employed. Each step of the process will occur sequentially notwithstanding the initial presence of all the reagents. Additional materials may be added as necessary. After the appropriate length of time has passed to produce the desired amount of the specific nucleic acid sequence, the reaction may be halted by inactivating the enzymes in any known manner or separating the components of the reaction.

Synthetic DNA sequences amplified by PCR generally contain mutations. The _Thermus aquaticus_ polymerase used in PCR produces single base substitution errors at a rate of 1 per 9,000 nucleotides polymerized.

In addition, frameshift errors (deletions and insertions of nucleotides) are produced at a rate of 1 per 41,000. For the synthesis of a 400 bp dsDNA sequence, roughly 1 base substitution mutation occurs per 11 cycles of PCR (800 nucleotides synthesized per cycle). Approximately 30 cycles of PCR are usually required for the amplification of a given DNA target sequence. Therefore, about 2-3 base substitution mutants and possibly 1 frameshift mutant might be produced upon synthesis of a 400 bp gene. The situation is somewhat more complicated (and deleterious) due to the propagation (amplification) of mutations made in early PCR steps.

The mutations which arise from PCR amplification can be easily corrected by designing the targeted dsDNA sequence with restriction ends for easy ligation into a plasmid containing an ssDNA phage origin of replication. This allows for characterization of the mutations by DNA-sequencing, and correction by site-directed mutagenesis.

Thus, this method involves: (1) PCR amplification of long DNA oligonucleotides corresponding to the opposing top and bottom strands of a target dsDNA sequence, yielding a dsDNA sequence which is likely to contain mutations; (2) cloning this dsDNA sequence into a plasmid containing a phage origin of replication such as f1(IG); and (3) fixing the mutations by site-directed mutagenesis to yield the correct double-stranded DNA sequences.

Site-directed mutagenesis is used to correct any mutations revealed by the sequencing carried out on the plasmid into which the gene was inserted in step E) of the method of this invention. The mutagenesis was carried out as follows.

1) transferring the plasmid into an E.coli strain that

    i) is deficient in dUTPase and uracil N-glycosylase and

    ii) includes a F′ element;

2) infecting E.coli resulting from 1) with a f1 helper phage (f1) thereby forming a single-stranded DNA version of the plasmid of 1) containing the f1 phage intergenic region and uracil bases (ssP);

3) isolating single-stranded plasmid (ssP) containing uracil and f1 phage intergenic region [fl-(IG)J secreted by the infected E.coli.;

4) annealing on each flank of the mutation a mutagenic oligonucleotide ( oligo) i) having DNA sequences that are complementary to those of plasmid flanking the mutation;

5) treating the plasmid of 4) with DNA polymerase and dATP, dTTP, dCTP and dGTP to synthesize a second DNA strand complementary to the plasmid of 4) resulting in the formation of a double-stranded plasmid;

6) infecting a wild-type E.coli strain with the plasmid of 5) thereby forming a single-stranded plasmid bearing a corrected gene sequence; and

7) converting the single-stranded plasmid of 6) to a double-stranded form using conventional cloning techniques as described in step 5) above.

Example of the Method - Design and Synthesis of the HIV-1 REV Gene

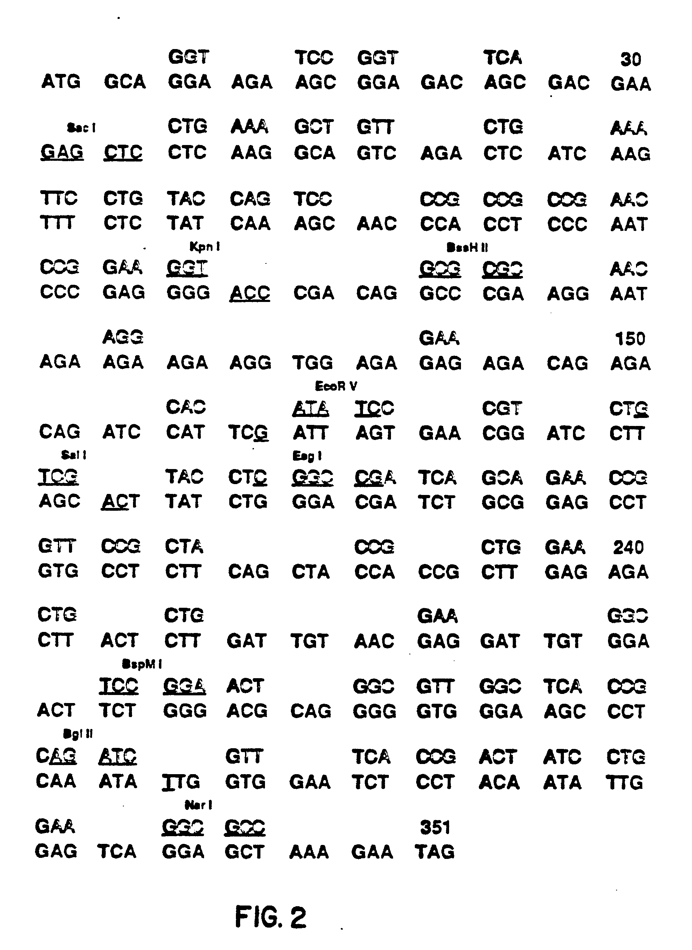

The nucleotide sequence of the native REV gene (formally ART or TRS) from HIV-1 isolate BH10 and the nucleotide sequence of the newly designed synthetic REV gene is given in Figure 2. All codon changes from the native gene to the synthetic gene are shown in open type. Unique restriction sites (Sac I, Kpn I, BssH II, EcoR V, Sal I, Eag I, BspM II, Bg II and Nar I) were incorporated into the gene by alternative codon usage. Other alternative codons were chosen to match the codons preferred by E.coli for protein expression purposes. Translation of either the native or synthetic REV genes using the genetic code gives exactly the same protein sequence.

The synthetic REV gene was designed with extra sequences flanking the 5' and 3' ends of the gene. Such extra sequences contained the restriction enzyme sites BamHI at the 5' end and EcoR1 at the 3' end. These sites were designed into the gene so that the PCR-amplified gene could be easily cloned into a plasmid such as pKH7 or M13 mp9 containing a phage origin of replication [f1(IG)-]. Any vector comprising a phage origin of replication would be useful in correcting mutations by site-directed mutagenesis. These restriction sites are also useful for cloning the gene into plasmids used for gene characterization (by DNA sequencing) and for protein expression.

The synthesis of the REV gene was carried out as follows. Both top and bottom strands of the complete REV gene, including 5' and 3' flanking sequences containing restriction sites BamHI and EcoR1 (not shown), were synthesized using standard reagents and techniques on a Biosearch automated DNA synthesizer, using 0.2 μmole DNA columns.

The efficiency of DNA synthesis was measured by standard techniques to be >98% per base addition. It was estimated that approximately 6 nmoles of each full length 393mer (both top and bottom strands) were made. DNA synthesis primers (20mers) complementary to the 3' ends of each strand were also synthesized.

The crude synthesis broths were desalted by gel filtration chromatography. The crude DNA synthesis products were present in pure water in a volume of 1.5 mL after this step. Aliquots (10 μL) of each of these DNA solutions, containing the two 393mers (top and bottom strands) and their failure sequences, were mixed together in a microtube, along with the two DNA synthesis primers (10 μM), and 10 mM Tris-Cl, ps 8.4, 2.5 mM MgCl2, 50 mM KCl and 1.0 mM of each dNTP (dATP, dCTP,dGTP, dTTP) and 10 units of tag polymerase, in total volume of 100 μl.

The microtube was placed in a programmable heating block at 70°C. The sample was then heated from 70°C-95°C over one minute to denature DNA, annealed at 55°C and then followed by a heating step at 70°C for 20 minutes for the first three cycles for tag polymerase catalyzed polymerization of the DNA. This procedure was repeated for an additional 32 PCR cycles. An aliquot

(10 μL) of this PCR reaction mix was placed in a second microtube, and buffer components and tag polymerase were added as above. PCR was carried out on this mixture for an additional 30 cycles.

After PCR amplification, approximately 10 μl of the PCR reaction mixture was characterized by DNA electrophoresis on 1% agarose gels, and a DNA band of approximately 400 bp was observed. Therefore, the remaining PCR reaction mixture was treated with 2.5 volumes of 95% ethanol at -20°C for minutes. The resulting precipitated DNA was collected by centrifugation at 10,000 x g. This DNA was dissolved in buffer and was separated by preparative DNA electrophoresis on a 1% agarose gel. The 400 bp DNA band was visualized by ethidium bromide staining. A slice was made in the gel in front of the visualized DNA band with a sharp razor blade. A small piece of DEAE-cellulose DNA binding paper was inserted into the slice in the gel. DNA electrohporesis was continued until all of the 400 bp DNA was bound to the paper.

The DNA was then eluted from the paper by treatment with 2 M NaCl at 65°C. Ethidium bromide was removed from the DNA by extraction with n-butanol. The DNA was collected by precipitation and centrifugation as before. The DNA pellet was washed with 70% ethanol in water. The purified 400 bp DNA fragment was redissolved in 10 μl of restriction enzyme buffer containing 0.5 μg plasmid pKH7, BamHI (10 U) and EcoR1 (10 U), and the digestion was carried out for 60 minutes at 37°C. This solution was extracted once with phenol:chloroform:isoamyl alcohol (24:24:1). The DNA, including pKH7 (Figure 1), was then precipitated as before. The DNA was dissolved in buffer containing 400 U of DNA ligase. Ligation was carried out at 13°C for 5 hours. The ligation mix was used to transfect E.coli strain TBI. Transfectants were selected as white colonies on XGAL plates. Plasmids were prepared from several colonies by conventional miniprep procedure, and then digested with Puvll.

Plasmid pKH7 contains two Puvll sites 300 bp apart. pKH7 also contains a bacterial origin of replication and a phage origin of replication. Since insertion of the 400 bp REV gene occurs between these two sites, plasmids containing the insertion should yield larger fragment of 700 bp (instead of 300 bp) following Puvll digestion. The Puvll digests of 18 plasmids were separated on 1% agarose gels, and 11/18 plasmids yielded 700 bp fragments.

Eight of these plasmids containing the 700 bp Puvll fragment were further digested with Sac I, EcoR V and Bgl II. These enzymes should only cut plasmids containing the REV gene. EcoR V and Bgl II should cut only once. Bence each cut should linearize the plasmid. Sac I should cut near the beginning and end of the gene and should yield a 350 bp fragment. The restriction digests of these plasmids were separated on 1% agarose gels. Plasmids containing the 700 bp Puv II fragment a ) were linearized with EcoR V and Bgl II, and b) yielded a 350 bp fragment with SaC I.

Both strands of the REV insert therein were analyzed by DNA sequencing. This confirmed that the REV gene had been completely synthesized. Bowever, several base substitution mutations in the nucleotide sequence were observed. These mutations included a C → T base substitution at residue 103, aT → C base substitution at residue 181, a T base deletion (frameshift) at residue 186 and G base insertion (frameshift) at residue 235.

A DNA oligonucleotide was synthesized corresponding to the 20 bases (residues 91-110) around the mutation at position 103. The plasmid PREVI was isolated as a single-stranded template containing deoxyuracil. Site-directed mutagenesis was carried out in vitro in order to correct the position 103 mutation. The other mutations were similarly corrected by site-directed mutagenesis. DNA sequencing following mutagenesis was carried out confirming that the final REV sequence was correct.

## Claims

1. A method of making targeted double-stranded DNA sequences characterized by the steps of:
   A) preparing first and second synthesis broths, seach comprising
   i) DNA oligonucleotides of a defined nucleotide length and failure sequences thereof; or
   ii) the failure sequences only;
   wherein the first synthesis broth comprises DNA oligonucleotides that include the top strand or failure sequences thereof of the targeted double-stranded DNA sequence and the second synthesis broth comprises DNA oligonucleotides that include the bottom strand or failure sequences thereof of the targeted DNA sequence;
   B) mixing the first and second synthesis broths together;
   C) adding DNA oligonucleotide primers that are complementary to the 3′ ends of the DNA oligonucleotides of A);
   D) applying polymerase chain reaction (PCR) procedures to the admixture of C) thereby amplifying exponentially the concentration of the target double-stranded DNA sequence;
   E) isolating the target double-stranded DNA sequence;
   F) cloning the target double-stranded DNA sequence in a cloning vector having a phage origin of replication;

G) sequencing the target double-stranded DNA sequence to identify mutations; and
   H) repairing the mutations by site-directed mutagenesis.

2. A method of making targeted double-stranded DNA sequences having more than 400 base pairs characterized by the steps of:
   A) preparing a series of first and second synthesis broths, each comprising;
   i) DNA oligonucleotides of a defined nucleotide length and failure sequences thereof; or
   ii) the failure sequences only;
   wherein
      a) the first broth of each serial member of the series comprises DNA oligonucleotides equivalent to a defined portion of the top strand or failure sequences thereof of the targeted double-stranded DNA sequence;
      b) the second broth in each serial member of the series comprises DNA oligonucleotides equivalent to a defined portion of the bottom strand or failure sequences thereof of the targeted double-stranded DNA sequence; and
      c) the entire series, taken together, is equivalent to the entire targeted double-stranded DNA sequence;
   B) separately mixing the first and second synthesis broths of each member of the series together;
   C) adding to each mixture of the series DNA oligonucleotide primers that are complementary to the 3′ ends of the DNA oligonucleotides in each mixture;
   D) applying polymerase chain reaction procedures to each mixture thereby amplifying exponentially the concentration of the targeted double-stranded DNA sequence equivalents in each mixture;
   E) isolating the targeted double-stranded DNA sequence equivalents from each mixture in the series;
   F) cloning each of the equivalents in a cloning vector having a phage origin of replication;
   G) sequencing each equivalent to identify any mutations;
   H) repairing any mutations by site-directed mutagenesis;
   I) removing each equivalent of the series from the cloning vector;
   J) ligating the entire series of equivalents together to form the targeted double-stranded DNA sequence having more than 400 base pairs.

3. The method of claim 1 or 2 wherein the initial cycles of the polymerase chain reaction are carried out over a period of time to allow hybridization of failure sequences.

4. The method of claim 3 wherein the first two cycles of the polymerase chain reaction are carried out over a period of greater than 1 minute.

5. The method of claim 3 wherein the first two cycles of the polymerase chain reaction is carried out over a period of at least 30 minutes.

6. The method of any one of the preceding claims wherein only failure sequences of the targeted double-stranded DNA sequence equivalents are present in the broths.

7. The method of claim 1 wherein the targeted double-stranded DNA sequence is a gene comprising up to 400 base pairs.

8. The method of any one of the preceding claims wherein the DNA oligonucleotide synthesis is carried out on an automated oligonucleotide synthesizer.

# FIG. 1

5' ——————————————————— 3'   400 bp gene (n)
3' ——————————————————— 5'

n-1  3' ———————— 5'        ———————— n-5
n-2     ————————             ———————— n-4
n-3     ————————             ———————— n-3      (FAILURE
n-4     ————————             ———————— n-2      SEQUENCES)
n-5     ————————             ———————— n-1

BOTTOM STRAND          TOP STRAND

↓ DNA primers (3'ends)

P⌐
3' ———————— 5'        ————————
   ————————             ————————
   ————————             ————————
   ————————             ————————
   ————————        5' ———— 3'
                        ⌐P

↓ PCR

5' ——————————————————— 3'
3' ——————————————————— 5'

↓ Purify, clone in
  plasmid with phage
  origin

                    gene
      pkH7

↓ sequence gene;
  repair mutations by site-directed
  mutagenesis

Complete Synthetic Gene

11

```
              GGT             TCC   GGT         TCA         30
    ATG   GCA GGA   AGA   AGC GGA   GAC   AGC   GAC   GAA

     Sec I        CTG   AAA   GCT   GTT         CTG         AAA
    GAG   CTC   CTC   AAG   GCA   GTC   AGA   CTC   ATC   AAG

    TTC   CTG   TAC   CAG   TCC         CCG   CCG   CCG   AAC
    TTT   CTC   TAT   CAA   AGC   AAC   CCA   CCT   CCC   AAT
                Kpn I                         BssH II
    CCG   GAA   GGT                     GCG   CGC         AAC
    CCC   GAG   GGG   ACC   CGA   CAG   GCC   CGA   AGG   AAT

          AGG                           GAA               150
    AGA   AGA   AGA   AGG   TGG   AGA   GAG   AGA   CAG   AGA
                            EcoR V
                CAC         ATA   TCC         CGT         CTG
    CAG   ATC   CAT   TCG   ATT   AGT   GAA   CGG   ATC   CTT
     Sal I                  Esp I
    TCG         TAC   CTC   GGC   CGA   TCA   GCA   GAA   CCG
    AGC   ACT   TAT   CTG   GGA   CGA   TCT   GCG   GAG   CCT

    GTT   CCG   CTA               CCG         CTG   GAA   240
    GTG   CCT   CTT   CAG   CTA   CCA   CCG   CTT   GAG   AGA

    CTG         CTG                     GAA               GGC
    CTT   ACT   CTT   GAT   TGT   AAC   GAG   GAT   TGT   GGA
          BspM I
          TCC   GGA   ACT         GGC   GTT   GGC   TCA   CCG
    ACT   TCT   GGG   ACG   CAG   GGG   GTG   GGA   AGC   CCT
          Bgl II
    CAG   ATC         GTT         TCA   CCG   ACT   ATC   CTG
    CAA   ATA   TTG   GTG   GAA   TCT   CCT   ACA   ATA   TTG
                Nar I
    GAA         GGC   GCC               351
    GAG   TCA   GGA   GCT   AAA   GAA   TAG
```

FIG. 2